# FASCICULE DE BREVET EUROPEEN

(11) **EP 3 125 999 B1**
(45) Date de publication et mention de la délivrance du brevet: **14.07.2021**
(21) Numéro de dépôt: 15715352.9
(22) Date de dépôt: 19.03.2015
(51) Int. Cl.: A61N 5/06, A61H 15/02, A61H 15/00

(54) **APPAREIL DE SOIN AVEC GUIDE DE LUMIERE**
BEHANDLUNGSVORRICHTUNG MIT EINEM LICHTLEITER
TREATMENT APPARATUS WITH A LIGHT GUIDE

(30) Priorité: 21.03.2014 FR 1452417
(43) Date de publication de la demande: 08.02.2017
(73) Titulaire: SEB S.A., 69130 Ecully (FR)
(72) Inventeur: GIRAUD, Camille, 69001 Lyon (FR); GAILHARD, Thierry, 69970 Chaponnay (FR)
(74) Mandataire: SEB Développement
(86) Numéro de dépôt international: PCT/FR2015/050682
(87) Numéro de publication internationale: WO 2015/140474

(56) Documents cités:
- WO-A1-03/103523
- WO-A1-2008/131079
- WO-A1-2012/133011
- DE-C1- 3 905 517
- US-A1- 2005 234 527
- US-A1- 2005 234 527
- US-A1- 2009 299 236
- US-A1- 2012 289 885

## Description

La présente invention concerne un appareil de soin de la peau, en particulier de la peau du visage, destiné à fournir des soins sous formes variées pour produire un effet coup d'éclat, remodelage, anti-âge ou antirides. Les soins sont procurés par un apport d'énergie lumineuse à la peau voire également par une action mécanique de moyens massants.

On connaît un document KR20090001911U qui enseigne un appareil de massage comprenant une tête de soin comprenant une chambre sous dépression destinée à aspirer la peau. La chambre intègre deux rouleaux parallèles qui sont motorisés de sorte à tourner dans le même sens. L'appareil comprend encore un émetteur de lumière dans le fond de la chambre derrière lesdits rouleaux. La source lumineuse est du type diode électroluminescente, communément appelée LED, destinée à projeter de la lumière simultanément au mouvement des rouleaux. L'inconvénient d'un tel agencement de la source lumineuse est qu'elle est éloignée de la zone à projeter, c'est-à-dire du plan tangent à l'extérieur des rouleaux, ce qui fait que l'intensité de la lumière projetée est diminuée. De plus, une partie des faisceaux lumineux est dissimulée par les deux rouleaux, ce qui réduit l'efficacité du traitement par la lumière.

On connaît un autre document JP3155121U qui décrit un appareil de massage portable comprenant un disque de traitement entraîné en rotation par un arbre de sortie moteur destiné à venir en contact avec la peau. L'appareil comprend en-dessous dudit disque une pluralité de sources lumineuses sous forme de LED destinées à éclairer la peau au travers dudit disque qui est fait en matière translucide. Le traitement par la projection des lumières et le traitement par le mouvement du disque se font de façon simultanée afin d'atteindre un meilleur résultat. Selon ce document, les LED sont agencées en cercle face au disque de traitement pour émettre des faisceaux lumineux perpendiculairement à la peau. Néanmoins, un tel agencement ne permet pas une projection homogène ou nécessite un nombre élevé de LED pour pouvoir couvrir toute la surface de projection, ce qui augmente le coût de l'appareil. Par ailleurs, le rendement de l'énergie lumineuse n'est pas optimisé, car la perte d'énergie est importante lorsque la lumière traverse le disque translucide. On connait également un autre document WO2012133011 relatif à un appareil de massage équipé d'un rouleau et d'une source lumineuse. Un appareil de soin de la peau comprenant une source lumineuse et un guide de lumière est connu de US 2012/289885 A1.

Le but de la présente invention est de remédier au moins en partie aux inconvénients précités et de proposer un appareil de soin de la peau permettant de fournir au moins un traitement mécanique combiné à un traitement par lumière sur une zone définie.

Un autre but de l'invention est un appareil de soin de la peau permettant une diffusion de lumière homogène sur la zone.

Un autre but de l'invention est un appareil de soin de la peau permettant un éclairage de la peau intense et efficace sur la zone.

Un autre but de l'invention est un appareil de soin de la peau dont les sources lumineuses permettent d'éclairer au moins une partie d'une zone sollicitée par des moyens de massage mécaniques auxquels elles sont associées.

Un autre but de l'invention est un appareil de soin de la peau avec un agencement des sources lumineuses et des moyens de massage mécaniques optimisé pour un encombrement de l'appareil réduit.

Encore un autre but de l'invention est un appareil de soin de la peau multifonctionnel, facile à utiliser et peu coûteux.

Ces buts sont atteints avec un appareil de soin pour la peau selon la revendication 1 comprenant un corps comportant un boîtier formant une zone de préhension, des moyens d'alimentation électrique agencés dans ledit corps, au moins une tête de soin, au moins une source lumineuse, au moins un guide de lumière présentant une surface de projection destinée à recevoir de la lumière émise par ladite au moins une source lumineuse et à projeter au moins un faisceau lumineux à destination d'une zone sur la peau. Selon l'invention, ledit au moins un guide de lumière comprend une face opposée à ladite surface de projection, ladite face opposée comprenant des moyens de distribution destinés à distribuer la lumière le long de ladite surface de projection. On comprend que le guide de lumière est fait en une pièce monobloc présentant à la fois la surface de projection et la face opposée qui est en regard, ce qui permet de diminuer le nombre de pièces et de réduire le coût de fabrication tout en gardant une bonne efficacité de concentration de lumière. Selon les différents modes de réalisation de l'invention, la tête de soin est conçue pour apporter des soins par illumination éventuellement aussi par action mécanique (massage).

Avantageusement, lesdits moyens de distribution comprennent au moins une surface (S1, S2) au moins partiellement réfléchissante destinée à réfléchir une partie de la lumière vers ladite surface de projection. La surface au moins partiellement réfléchissante fait partie de la face opposée et permet de réfléchir au moins une partie de la lumière projetée, la réflexion se faisant à l'intérieur du guide de lumière.

Afin de définir sa forme géométrique, ledit au moins un guide de lumière présente au moins une section transversale (S) de passage de la lumière qui est une section passant par un plan (P) perpendiculaire à ladite surface de projection, ladite section transversale (S) étant sécante avec la surface de projection et ladite au moins une surface (S1) respectivement suivant une première droite (D1) et une deuxième droite (D2).

Ainsi, ladite au moins une source lumineuse est agencée à une extrémité de ladite section transversale (S) entre la première droite (D1) et la deuxième droite (D2). Ainsi, la lumière traverse une paroi du guide de lumière agencée entre la surface de projection et la face opposée.

Selon une variante du guide de lumière, la distance absolue entre la première droite (D1) et la deuxième droite (D2) est constante depuis l'extrémité où est agencée ladite au moins une source lumineuse. Ceci représente un guide de lumière sous forme d'un plateau sensiblement plan.

Selon une autre variante du guide de lumière, la distance absolue entre la première droite (D1) et la deuxième droite (D2) est décroissante depuis l'extrémité où est agencée ladite au moins une source lumineuse. Les surfaces réfléchissantes sont alors agencées successivement sur une pente, chaque surface recevant au moins une partie de la lumière émise grâce à la réduction de la section transversale au droit de chaque surface à mesure que l'on s'éloigne de la source lumineuse.

De surcroît, lesdits moyens de distribution comprennent au moins deux surfaces (S1, S2) au moins partiellement réfléchissantes, dont une desdites deux surfaces (S2) et ladite section transversale (S) sont sécantes suivant une troisième droite (D3), la distance entre D1 et D2 étant supérieure à celle entre D1 et D3. Une telle section de passage correspond à un guide de lumière présentant des stries sous forme de rainures régulières sur la face opposée. Ceci permet de renforcer la réflexion de la lumière et de diminuer la réfraction au niveau de la face opposée. On obtient une diffusion encore plus homogène de la lumière le long de la surface de projection.

Ledit guide de lumière est un solide de révolution de ladite section transversale autour d'un axe central (Δ). Le guide de lumière présente alors une forme circulaire.

Avantageusement, la face opposée présente des cannelures partant dudit axe central (Δ). Ceci permet de renforcer la réflexion de la lumière et de diminuer la réfraction au niveau de la face opposée. Les faisceaux lumineux sont bien répartis sur la surface de projection circulaire, notamment les zones entre deux sources lumineuses voisines.

Alternativement, la face opposée présente des creux en forme de demi-sphère, par exemple comme une balle de golf ou analogue. Ceci permet de faire varier l'angle de réflexion macroscopique des faisceaux lumineux et de les homogénéiser.

De surcroît, ladite face opposée est au moins partiellement recouverte d'une couche de couleur blanche. Ou alternativement, ladite face opposée est au moins partiellement recouverte d'une couche de couleur métallisée. Ceci permet de renforcer la réflexion et de diminuer la réfraction au niveau de la face opposée.

Avantageusement, ladite surface de projection présente des moyens diffusants tels que du grainage, par exemple de type chimique ou charmille. Ceci accentue la diffusion de la lumière et permet d'homogénéiser davantage les faisceaux sur la peau.

Avantageusement, ladite au moins une source lumineuse est une diode électroluminescente (LED), car une telle source lumineuse est à la fois intense et compacte et permet donc d'optimiser simultanément l'efficacité et l'encombrement de l'appareil.

Selon un deuxième mode de réalisation de l'appareil, ladite au moins une tête de soin comprend des moyens de massage mécanique destinés à venir en contact avec la peau et des moyens de manœuvre desdits moyens de massage actionnés par un moteur électrique qui est relié auxdits moyens d'alimentation électrique. La mise en œuvre de tels massages mécaniques permet d'apporter une stimulation de la circulation sanguine dans la peau du visage. Les cellules sont d'autant plus activées par le traitement de photothérapie simultanément appliqué au massage.

Selon une première variante du deuxième mode de réalisation, lesdits moyens de massage comprennent au moins une bille de massage destinée à être déplacée en rotation selon au moins un axe de rotation vertical qui est perpendiculaire à la surface de projection.

Selon cette variante, ledit guide de lumière présente au moins un trou traversant laissant passer ladite bille de massage. Ainsi, on arrive à combiner le soin par illumination avec le soin par action mécanique tout en gardant la compacité de l'appareil.

Ladite source lumineuse comprend une pluralité de diodes électroluminescentes réparties de façon régulière autour dudit guide de lumière pour une distribution homogène de la lumière.

Selon une deuxième variante du deuxième mode de réalisation, lesdits moyens de massage comprennent un élément d'appui destiné à venir contre le visage et deux doigts de massage destinés à venir au contact de la peau de façon alternative.

Selon cette variante, ledit guide de lumière est agencé à proximité dudit élément d'appui et sensiblement parallèle à celui-ci.

Ainsi, ladite source lumineuse comprend au moins une diode électroluminescente entre lesdits doigts de massage et le corps.

Selon une troisième variante du deuxième mode de réalisation, lesdits moyens de massage comprennent deux rouleaux de massage qui sont mobiles en rotation sur eux-mêmes selon deux axes de rotation horizontaux (B1, B2) parallèles entre eux et chacun perpendiculaire à l'axe du corps, un premier rouleau comprenant au moins une palette s'étendant radialement en saillie de la surface du premier rouleau et le deuxième rouleau présentant une surface de type lisse, lesdits rouleaux étant actionnés par des moyens de manœuvre.

Selon cette variante, l'appareil comprend au moins un guide de lumière destiné à recouvrir au moins une zone de contact (Z) à l'extrémité des rouleaux destinée à venir en contact avec la peau.

Ainsi, ladite source lumineuse comprend au moins une diode électroluminescente à proximité de ladite zone de contact (Z).

Selon une quatrième variante du deuxième mode de réalisation, lesdits moyens de massage comprennent deux rouleaux cylindriques qui sont mobiles en rotation sur eux-mêmes selon deux axes de rotation (C1, C2) parallèles entre eux et par rapport à l'axe du corps.

Selon cette variante, l'appareil comprend au moins un guide de lumière destiné à recouvrir au moins une première zone d'éclairage (Z') à l'une des extrémités des rouleaux cylindriques et/ou une deuxième zone d'éclairage (Z") située entre lesdits rouleaux cylindriques, ladite première zone d'éclairage (Z') étant destinée à venir en contact avec la peau.

Ainsi, ladite source lumineuse comprend au moins une diode électroluminescente à proximité de ladite première zone d'éclairage (Z').

Avantageusement, lesdits moyens de massage sont transparents ou translucides de sorte à permettre la transmission de la lumière.

De surcroît, ladite au moins une tête de soin est détachable dudit corps et l'appareil comprend au moins deux têtes de soin différentes interchangeables sur le corps.

L'invention sera mieux comprise à l'étude des modes de réalisation pris à titre nullement limitatif et illustrés par les dessins annexés dans lesquels :
- La figure 1 illustre une vue d'un premier mode de réalisation de l'appareil selon l'invention,
- La figure 2 illustre une vue de la tête de soin selon le premier mode de réalisation,

- La figure 3 illustre une vue de l'agencement des sources lumineuses dans la tête de soin selon le premier mode de réalisation,
- Les figures 4 à 9 illustrent différentes variantes du guide de lumière selon le premier mode de réalisation,
- Les figures 10 à 13 illustrent différentes sections de passage de la lumière selon le premier mode de réalisation,
- Les figures 14 à 18 illustrent des vues d'un deuxième mode de réalisation de l'appareil selon l'invention, selon une première variante,
- Les figures 19 et 20 illustrent des vues du deuxième mode de réalisation de l'appareil, selon une deuxième variante,
- Les figures 21 et 22 illustrent des vues du deuxième mode de réalisation de l'appareil, selon une troisième variante,
- Les figures 23 à 25 illustrent des vues du deuxième mode de réalisation de l'appareil, selon une quatrième variante,
- La figure 26 illustre une vue du guide de lumière selon la troisième variante du deuxième mode de réalisation de l'appareil,
- La figure 27 illustre une vue du guide de lumière selon la quatrième variante du deuxième mode de réalisation de l'appareil.

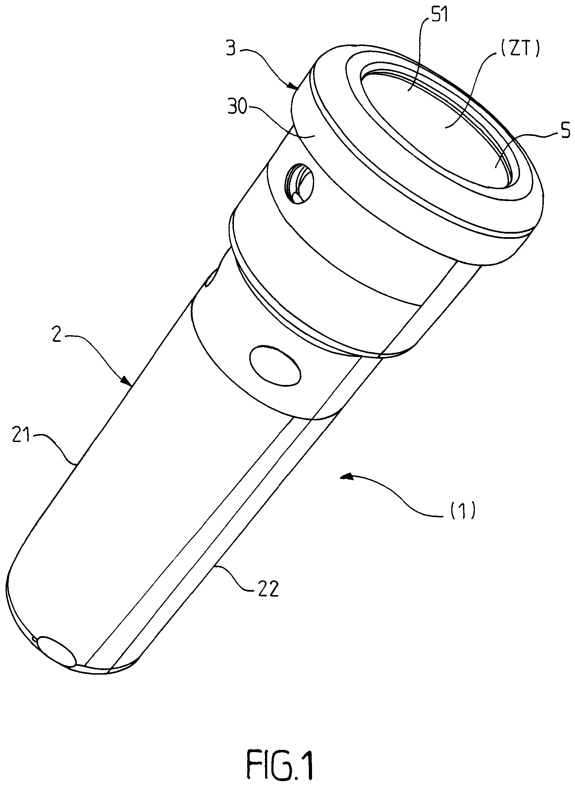

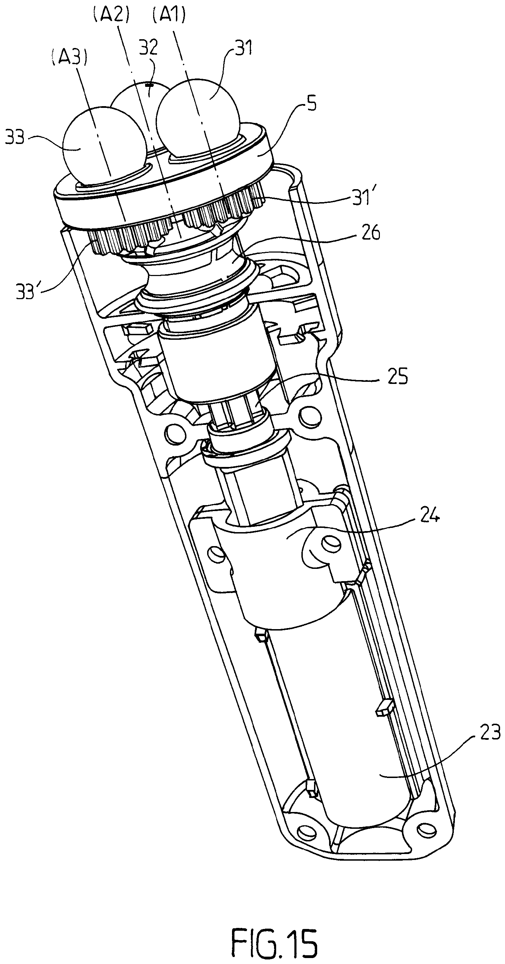

Comme illustré en figure 1 l'invention concerne un appareil de soin 1 pour la peau comprenant un corps 2 d'une forme longitudinale et d'un axe central (Δ) comportant un boîtier constitué de deux demi-boîtiers 21, 22 formant ensemble une zone de préhension. Une tête de soin 3 est montée sur une des extrémités du corps présentant une zone de traitement (ZT) destinée à fonctionner sur ou près de la peau. Ladite zone de traitement (ZT) peut être une zone de projection de lumière et/ou une zone présentant des moyens de massage mécanique suivant les modes de réalisation. L'appareil de soin 1 comprend encore à l'intérieur du corps 2 des moyens d'alimentation électrique 23 tels que visibles à la figure 15 comme par exemple une batterie rechargeable.

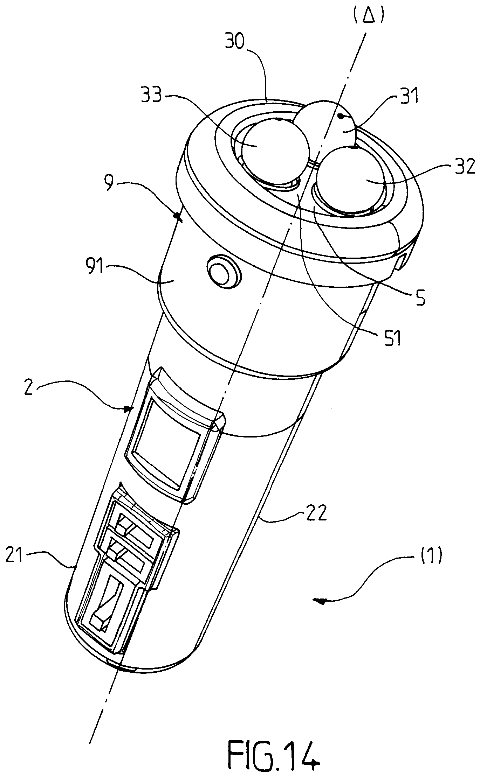

L'invention sera décrite en deux parties avec
- une première partie concernant le premier mode de réalisation de l'appareil dépourvu de moyen de massage mécanique, qui est illustré par les figures 1 à 13;
- une deuxième partie concernant le deuxième mode de réalisation de l'appareil dans ses différentes variantes, le deuxième mode de réalisation comprenant des moyens de massage mécaniques qui sont illustrés par les figures 14 à 27.

### Partie I

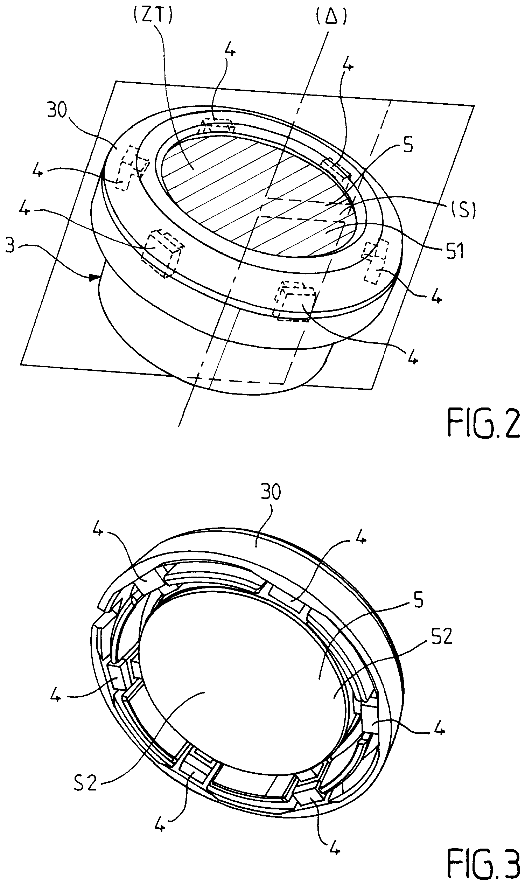

Tel qu'illustré aux figures 2 et 3, la tête de soin 3 présente une forme substantiellement cylindrique d'axe central (Δ) et comprend une pluralité de sources lumineuses 4 qui sont des LED agencées à l'intérieur du cylindre. Selon l'exemple illustré, les LED 4 sont au nombre de six et sont réparties de façon régulière sur le pourtour du cylindre. Les LED 4 sont alimentées par les moyens d'alimentation électrique 23 au moyen de fils électriques (non illustrés) de façon à ce que chaque LED soit orientée vers le centre du cylindre et projette des faisceaux lumineux dans cette direction. En ce qui concerne le positionnement des LED 4, la tête de soin 3 comprend une couronne 30 présentant des cavités circonférentielles dans lesquelles sont encastrées les LED, ladite couronne 30 pouvant être transparente, translucide ou bien opaque. La couronne 30 a également pour but de dissimuler les LED pour qu'elles soient protégées de l'extérieur.

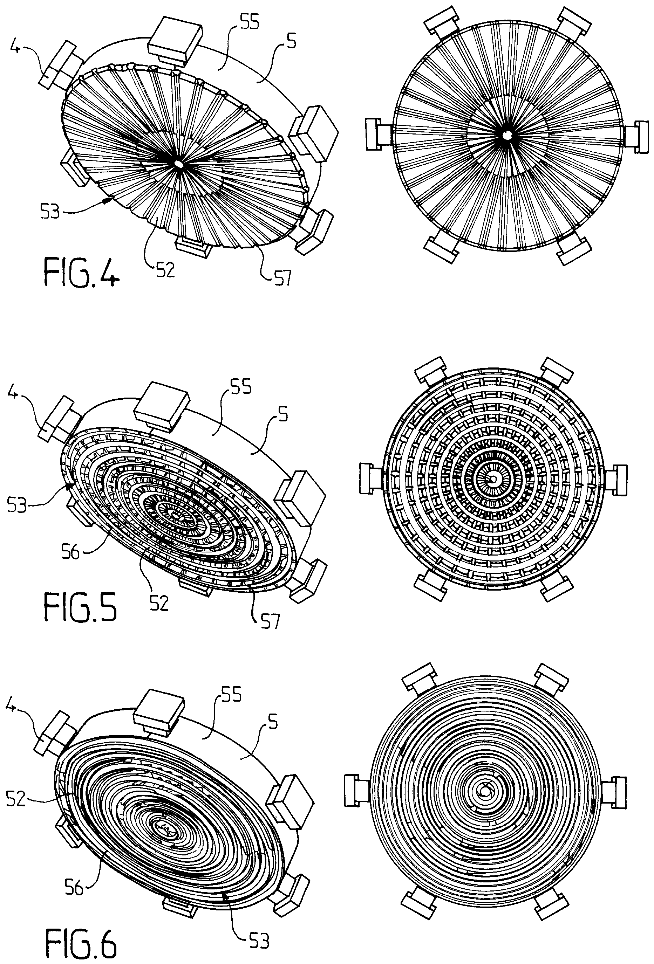

La tête de soin 3 comprend en son intérieur un guide de lumière 5 qui a pour objectif d'orienter et d'homogénéiser et de concentrer les faisceaux lumineux provenant des LED vers la peau. Pour ce faire, ledit guide de lumière 5 présente une forme de plateau sensiblement circulaire et monobloc ayant une surface extérieure lisse qui est une surface de projection 51 de la lumière. Ladite surface de projection 51 définit la zone de traitement (ZT) qui est destinée à venir près de la peau ou bien contre la peau. Le guide de lumière 5 présente encore une face opposée 52 qui est agencée en regard de ladite surface de projection 51 et écartée de celle-ci par une paroi latérale 55 sensiblement cylindrique visible sur la figure 4. Les LED sont donc réparties autour du guide de lumière 5 face à la paroi latérale 55.

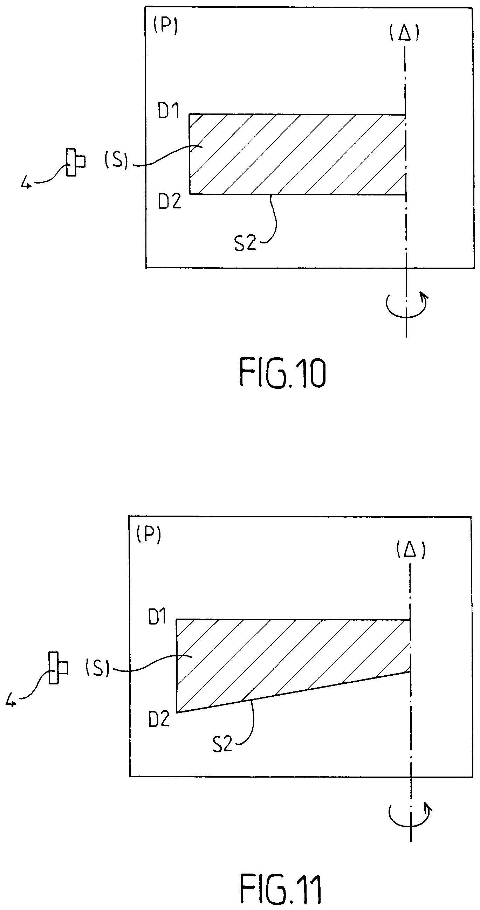

Ladite face opposée 52 comprend des moyens de distribution 53 destinés à distribuer la lumière le long de la surface de projection 51. Selon une première variante desdits moyens de distribution 53, il s'agit d'une surface S2 lisse et réfléchissante comme visible sur la figure 3 destinée à recevoir les faisceaux lumineux provenant des LED et à les réfléchir vers la surface de projection 51. Afin de mieux schématiser ceci, on pourrait imaginer une section transversale (S) de passage de la lumière vers le centre qui est obtenue par l'intersection d'un plan (P) perpendiculaire à ladite surface de projection 51, le guide de lumière 5 et l'axe central (Δ). Comme illustré à la figure 10, la section transversale (S) et la surface de projection 51 sont sécantes suivant une première droite D1 ; la section transversale (S) et la face opposée 52 sont sécantes suivant une deuxième droite D2. La distance entre D1 et D2 est constante depuis l'extrémité où est agencée la LED. Le guide de lumière 5 selon cette variante est donc un solide de révolution de ladite section transversale (S) autour de l'axe central (Δ).

Les moyens de distribution 53 pourraient également prendre d'autres formes différentes qui sont illustrées aux figures 4 à 9. Selon cette deuxième variante, les moyens de distribution 53 comprennent une série de surfaces réfléchissantes présentées sous formes de stries sur la face opposée 52.

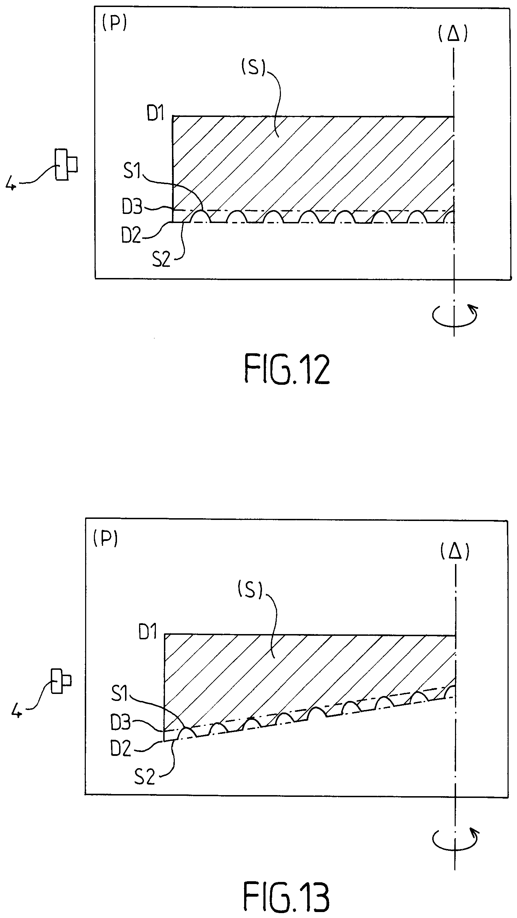

Selon une première variante des stries, les moyens de distribution 53 présentent sur la face opposée 52 des cercles creux 56 concentriques pour guider les faisceaux lumineux à parcourir toute la section transversale (S) jusqu'au centre du guide de lumière 5 afin d'avoir une répartition homogène de la lumière sur la surface de projection 51. Dans cette variante, la deuxième droite D2 représentant la partie non creuse de la face opposée 52 est discontinue. La section transversale (S) et le guide de lumière 5 sont sécants suivant une troisième droite D3 discontinue représentant la partie creuse de la face opposée 52, la distance entre D1 et D2 étant supérieure à la distance entre D1 et D3. La figure 12 montre la section transversale (S) constituant les cercles creux 56 par révolution autour de l'axe central (Δ), les variantes du guide de lumière 5 présentant de tels cercles sont illustrés à la figure 6.

Selon une deuxième variante des stries, les moyens de distribution 53 présentent sur la face opposée des cannelures 57 en rayons depuis l'axe central (Δ) vers la paroi latérale 55 afin de répartir les lumières sur la surface de projection 51 entre deux LED voisines, comme illustré à la figure 4.

Les cercles creux 56 concentriques et les cannelures 57 en rayons pourraient être combinés afin d'avoir une meilleure répartition de la lumière. Une telle combinaison permet de faire varier l'angle de réflexion macroscopique des faisceaux lumineux et de les homogénéiser. Un exemple du guide de lumière 5 combinant les deux moyens de distribution est illustré à la figure 5.

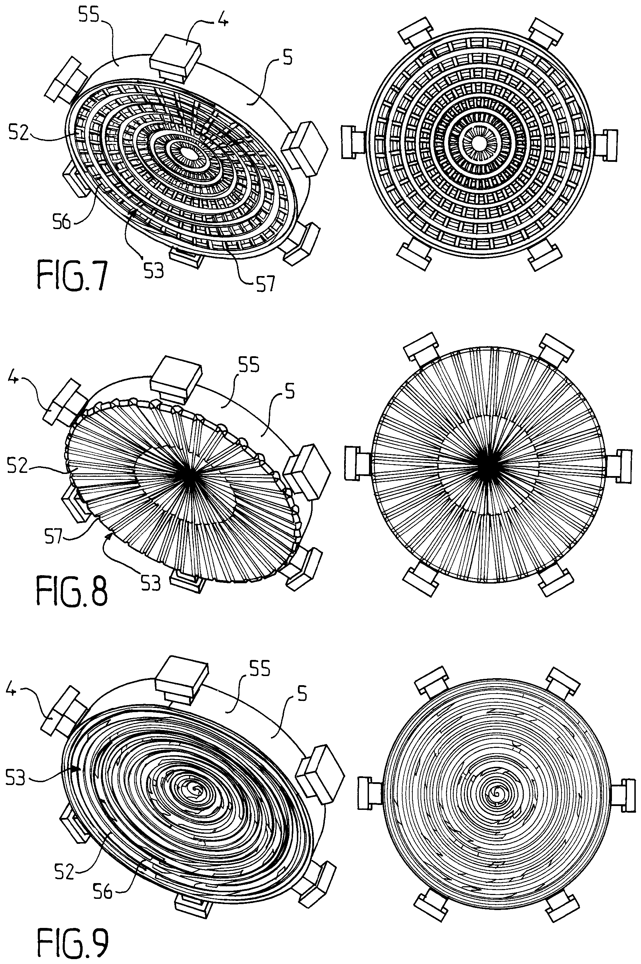

Toujours selon l'exemple du plateau circulaire, les moyens de distribution 53 peuvent comprendre aussi une surface conique creuse vers l'intérieur du plateau. Selon les variantes dans lesquelles les moyens de distribution 53 sont une surface lisse ou une surface présentant des cannelures 57, la face opposée forme une pente telle que visible à la figure 11 pour que toute la surface soit sollicitée pour réfléchir une partie de la lumière émise. Selon la variante dans laquelle les moyens de distribution comprennent des cercles creux 56, la distance entre D1 et D2 est décroissante depuis l'extrémité du guide de lumière 5 vers l'axe central (Δ) comme visible à la figure 13. Le guide de lumière selon cette variante est illustré à la figure 9. Ainsi, chaque strie est sollicitée par les faisceaux lumineux pour les renvoyer vers la surface de projection 51. La forme conique de la face opposée permet de mieux renvoyer et répartir les faisceaux lumineux vers la peau afin d'obtenir une meilleure efficacité de la lumière grâce à une plus forte intensité globale. Bien sur, le guide de lumière ayant une telle forme conique sur la face opposée pourrait présenter uniquement des cannelures 57 comme illustré à la figure 8, ou les deux types de stries tel que visible à la figure 7.

Le guide de lumière 5 peut être réalisé en matériau plastique du type PC (polycarbonate), PET (polyéthylène téréphtalate) incolore, SAN (styrène-acrylonitrile) ou thermoplastique du type PMMA (Poly méthacrylate de méthyle) qui présente un bon indice de milieu, une bonne réflexion aux interfaces et une bonne transparence pour moins d'absorption de l'énergie et donc moins de pertes. On pourrait aussi envisager d'autres matériaux, par exemple du verre.

Afin de masquer l'intérieur du guide de lumière et de rendre la projection du faisceau lumineux la plus homogène possible, la surface de projection 51 peut être dépolie ou grainée, par exemple avec un grainage de type chimique ou charmille.

### PARTIE II

Nous allons maintenant décrire le deuxième mode de réalisation de l'invention, dans lequel l'appareil comprend des moyens de massage 31, 32, 33, 34, 35, 36, 37, 37', 38, 39, 39' selon quatre variantes telles qu'illustrées aux figures 14 à 27.

Dans le deuxième mode de réalisation, chaque tête de soin 6, 7, 8, 9 comprend des moyens d'adaptation de la tête de soin 6, 7, 8, 9 sur le corps 2 de façon amovible, lesdits moyens d'adaptation 61, 71, 81, 91 étant formés par un fourreau permettant à la tête de soin 6, 7, 8, 9 d'être en partie engagée dans le corps 2. Le corps 2 comprend un moteur électrique 24 relié à un arbre de sortie 25 par des moyens de transmission, l'arbre de sortie 25 étant accessible au niveau de l'extrémité des moyens d'adaptation 61, 71, 81, 91. Le moteur électrique 24 est piloté par une unité de commande raccordée à une interface de commande manuelle accessible depuis l'extérieur du corps 2. L'interface de commande manuelle peut par exemple comprendre un interrupteur marche-arrêt et/ou des moyens de sélection manuelle de programmes de fonctionnement.

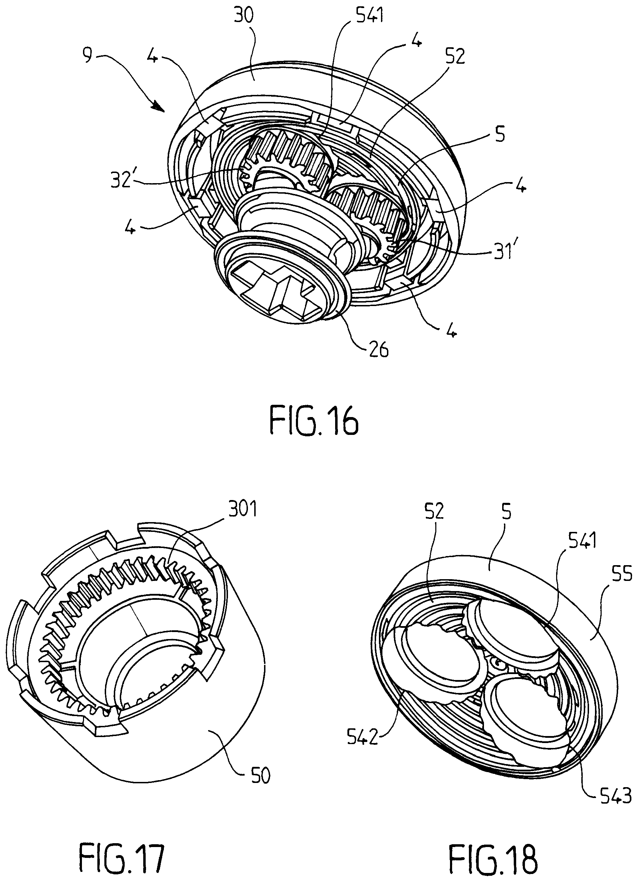

La tête de soin 9 selon la première variante telle qu'illustrée par les figures 14 à 18, présente une forme sensiblement cylindrique coaxiale avec le corps 2 de l'appareil. La tête de soin 9 comprend une couronne 30 d'appui délimitant une zone de travail à l'intérieur de laquelle se trouvent trois billes de massage 31, 32, 33 s'étendant en saillie du plan de la couronne 30, comme illustré à la figure 14. Selon l'exemple illustré, chaque bille de massage présente une surface de contact qui est déformable pour bien entrainer la peau avec le mouvement des billes. Par exemple, la bille peut être au moins partiellement en silicone pour un effet doux et accrochant. Comme illustré à la figure 15, chaque bille de massage 31, 32, 33 est reliée à des moyens de manœuvre pour être entrainée en rotation non seulement par rapport à l'axe central (Δ) mais aussi par rapport à un axe de rotation vertical A1, A2, A3 qui lui est propre. Pour ce faire, les moyens de manœuvre comprennent un entraineur 26 destiné à s'emboîter avec l'arbre de sortie 25 du moteur pour sa mise en mouvement. Par exemple, l'entraineur 26 peut présenter une cavité en forme de croix destinée à recevoir l'arbre de sortie 25 par une protubérance de la même forme sur celui-ci. L'entraineur 26 d'axe (Δ) porte sur une face supérieure trois pions (non illustrés) engagés chacun dans un alésage axial d'un pignon planétaire 31', 32', 33'. Chaque bille de massage est fixée sur le pignon planétaire correspondant. Chaque pignon planétaire engrène la périphérie dentée 301 d'une bague fixe 50, illustrées à la figure 17, de sorte que la rotation de l'entraineur 26 entraine le mouvement planétaire des billes de massage 31, 32, 33, c'est-à-dire un mouvement de rotation principal des trois billes autour de l'axe central (Δ) combiné à un mouvement de rotation secondaire de chacune des billes autour de l'axe de rotation vertical A1, A2, A3 qui se déplace en rotation autour de l'axe central (Δ) lors du fonctionnement de l'appareil.

Comme illustré aux figures 16 et 17, la tête de soin 9 comprend une série de LED agencées sur le pourtour de la couronne 30 comme dans le premier mode de réalisation. Le guide de lumière 5 selon cette variante est agencé entre la couronne 30 d'appui et la bague fixe 50. Tel que visible à la figure 18, le guide de lumière 5 présente des trous traversants 541, 542, 543 destinés à laisser passer les billes de massage 31, 32, 33. Le guide de lumière 5 est entraîné en rotation grâce au mouvement des billes.

La tête de soin 9 conformément à cette première variante permet de pétrir la peau du visage, notamment les zones larges telles que les joues ou le front, pour activer la microcirculation et stimuler les mécanismes naturels de production des protéines structurantes de la peau et ainsi prévenir les signes de l'âge. Ces effets sont accentués par la présence de la lumière sur la peau.

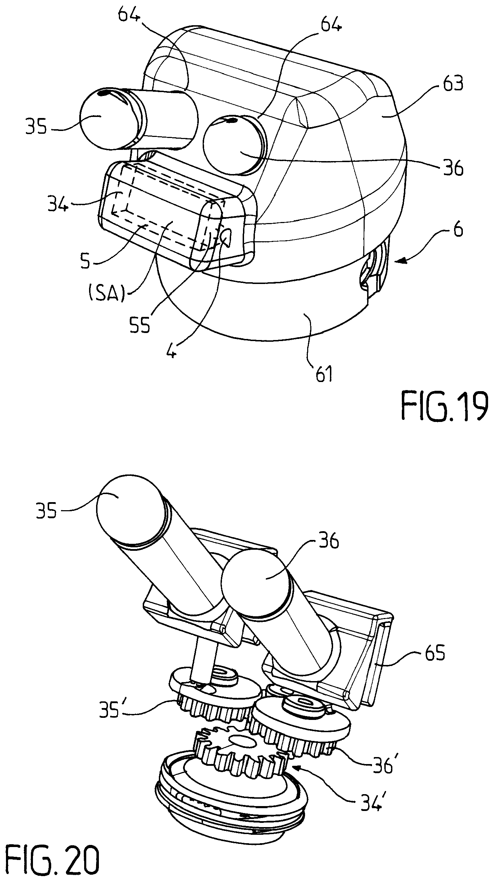

La tête de soin 6 selon une deuxième variante telle qu'illustrée aux figures 19 et 20 est conçue pour réaliser un massage par tapotement. À cet effet, la tête de soin 6 comprend un élément d'appui 34 qui est destiné à venir contre le visage pour définir une distance de travail. Ainsi, l'élément d'appui 34 définit une surface d'appui (SA) qui forme une surface de référence. La surface d'appui (SA) est idéalement lisse et elle peut présenter une forme concave qui lui permet de bien épouser la conformation des pommettes lorsque le massage est réalisé sous les yeux ou autour des yeux.

La tête de soin 6 comprend au-dessus de la surface d'appui (SA) et à l'opposé du corps 2 par rapport à ladite surface d'appui (SA), des doigts de massage 35, 36 qui comprennent chacun une tête de travail destinée à venir au contact du visage. Les deux doigts de massage 35, 36 sont chacun mobiles entre, d'une part, une position de rétraction correspondant à la position du doigt de massage 36 situé au premier plan à la figure 19 et, d'autre part, une position d'extension correspondant à la position du doigt de massage 35 situé au second plan à la même figure. En position de rétraction, la tête de travail de chaque doigt de massage 35, 36 se trouve en deçà de la surface d'appui (SA) vers l'intérieur de la tête de soin 6. Tandis qu'en position d'extension, la tête de travail de chaque doigt de massage 35, 36 se trouve au-delà de la surface d'appui (SA) vers l'extérieur de la tête de soin 6. Chaque tête de travail possède alors entre ses positions de rétraction et d'extension, une amplitude de déplacement comprise entre 5 mm et 15 mm. Par ailleurs, en position d'extension chaque tête de travail est saillante par rapport à la surface d'appui (SA) d'une distance comprise entre 2 mm et 10 mm.

La tête de soin 6 comprend également des moyens de manœuvre 34', 35', 36' adaptés pour déplacer alternativement chacun des doigts de massage 35, 36 entre ses positions d'extension et de rétraction. Les moyens de manœuvre 34', 35', 36' sont alors adaptés pour coopérer avec l'arbre de sortie 25 de manière à transmettre et à transformer le mouvement de rotation du moteur électrique 24 en un mouvement alternatif des doigts de massage 35, 36.

Selon l'exemple illustré, chaque doigt de massage 35, 36 est réalisé sous la forme d'une sorte de piston rectiligne qui s'étend en partie au moins à l'extérieur d'un corps creux 63 entourant les moyens de manœuvre 34', 35', 36'. Chaque doigt est alors guidé en translation par un alésage 64 aménagé dans le corps creux 63. L'extrémité de chaque doigt de massage 35, 36 située à l'intérieur du corps creux 63 coopère avec un pion excentré (non illustré) porté par un disque de manœuvre 35', 36' appartenant aux moyens de manœuvre. Le pion excentré est disposé dans une chambre 65 qui est liée rigidement au doigt de massage correspondant et dans laquelle le pion excentré peut se déplacer en translation de sorte que sa rotation avec le disque de manœuvre 35', 36' est transformée en une translation du doigt de massage 35, 36 correspondant.

On pourrait très bien imaginer d'autres modes de réalisation de la tête de soin 6 à tapotement, tels qu'un système bielle-manivelle ou encore un entrainement par came basculant les doigts de massage de façon alternative autour d'un axe.

Comme illustré à la figure 19, la tête de soin 6 selon la deuxième variante comprend au moins une LED 4 dans le corps creux 63 et à une extrémité de l'élément d'appui 34 de telle sorte que les faisceaux lumineux partent vers l'extrémité opposée. La tête de soin 6 comprend alors dans le corps creux 63 un guide de lumière 5 sensiblement rectangulaire qui correspond substantiellement à la surface d'appui (SA). La LED est donc agencée à l'extrémité du guide de lumière 5 en regard d'une paroi latérale 55 de celui-ci. La surface de projection 51 est voisine de la surface d'appui (SA) et la face opposée 52 peut être parallèle à la surface de projection 51 ou bien se rapprocher de la surface de projection 51 progressivement depuis l'extrémité où est présente la LED 4.

Afin de bien éclairer la peau, l'élément d'appui 34 est transparent ou translucide pour faire passer la lumière.

Un tel moyen de massage permet, par le tapotement assuré par les doigts de massage, de stimuler la circulation sanguine autour des yeux pour réduire les cernes et les poches. L'appareil de massage peut également être utilisé pour relancer le métabolisme, en particulier la production des éléments constitutifs de la peau, et ainsi traiter les ridules et les rides du visage, notamment au niveau du sillon naso-génien, en stimulant la circulation sanguine, ralentie par le plissement de la peau au niveau des rides. Ces effets sont accentués par la présence de la lumière sur la peau.

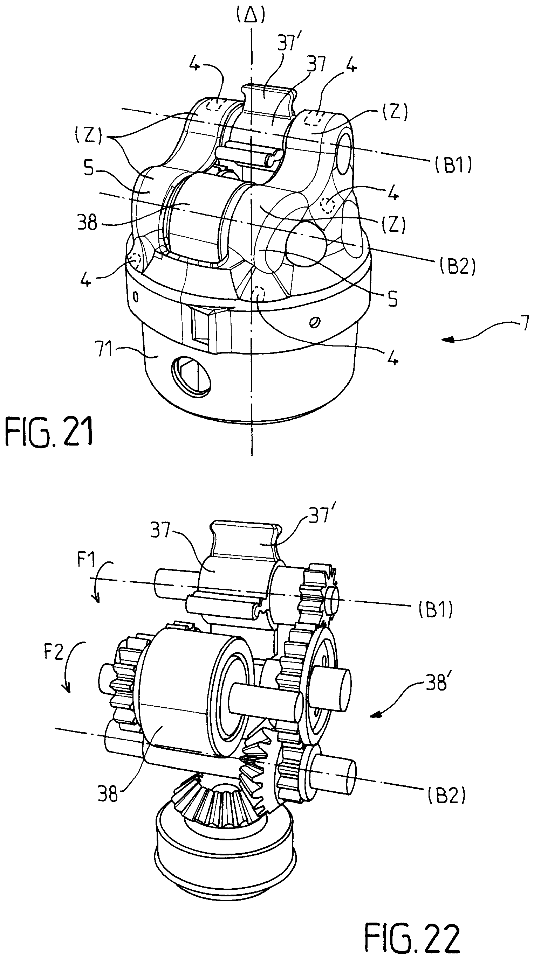

La tête de soin 7 selon la troisième variante telle qu'illustrée aux figures 21 et 22 est conçue pour réaliser un massage par pincement. À cet effet, la tête de soin 7 comprend, en tant que moyens de massage, deux rouleaux 37, 38 de massage qui sont mobiles en rotation sur eux-mêmes selon deux axes de rotation horizontaux B1 et B2 parallèles entre eux et perpendiculaire à l'axe central (Δ) tel que mieux visible à la figure 21. Les deux rouleaux 37, 38 de massage sont distants l'un de l'autre en étant séparés par une zone de travail. Selon l'exemple illustré, la distance entre les axes de rotation horizontaux B1 et B2 est constante.

Par ailleurs, selon l'exemple illustré, les rouleaux 37, 38 de massage sont agencés au sein de la tête de soin 7 de manière qu'un plan tangent aux deux rouleaux et situé vers l'extérieur de la tête de soin 7 forme avec un axe longitudinal du corps 2 un angle non nul et différent de l'angle droit.

Conformément à l'invention, un premier rouleau 37 comprend au moins une palette 37', et dans le cas présent représenté à titre d'exemple quatre palettes qui s'étendent radialement en saillie de la surface du premier rouleau 37. La tête de soin 7 présente de part et d'autre des rouleaux 37, 38 une zone de contact (Z) destinée à venir en appui contre la peau pour le maintien de l'appareil sur la peau pendant le traitement. La surface périphérique du premier rouleau 37 est alors située en retrait de la zone de contact (Z). Les palettes 37' présentent une extension radiale suffisante pour se trouver en saillie de la zone de contact (Z) au fur et à mesure de la rotation du premier rouleau 37. Les palettes 37' sont en outre réparties régulièrement à la périphérie du premier rouleau 37 et sont, dans le cas présent, placées à 90° les unes des autres.

Selon l'exemple illustré, les palettes 37' possèdent des sections droites transversales de formes différentes, étant entendu que toutes les palettes pourraient présenter une même forme. Ainsi, la palette 37' présente, vue en section droite transversale, une forme en « 8 ». D'autres formes de palette sont également envisageables telles qu'une palette qui possède, vue en section droite transversale, une extrémité libre plus épaisse que le reste, ou encore une palette qui possède, vue en section droite transversale, une forme droite, ou enfin, une palette qui possède, en section droite transversale, une forme allongée et effilée dont l'épaisseur se réduit en allant vers l'extrémité.

Le deuxième rouleau 38 présente une surface périphérique de type lisse qui s'étend en saillie de la zone de contact (Z) ou qui s'étend à flanc de la zone de contact (Z).

Selon l'invention, la tête de soin 7 comprend également des moyens de manœuvre 38' adaptés pour entrainer les rouleaux 37 et 38 dans le même sens, allant de l'extérieur de la zone de travail vers l'intérieur de la zone de travail pour le premier rouleau 37, et de l'intérieur vers l'extérieur de la zone de travail pour le second rouleau, vu depuis l'extérieur de la tête de soin et tel qu'indiqué par les flèches F1 et F2 sur la figure 22. Les moyens de manœuvre 38' sont alors adaptés pour coopérer avec l'arbre de sortie 25 de manière à transmettre et transformer le mouvement de rotation du moteur électrique selon l'axe central (Δ) en des mouvements de rotation selon les axes B1 et B2 qui présentent une direction orthogonale à celle de l'axe central (Δ).

Selon l'exemple illustré à la figure 22, les moyens de manœuvre 38' comprennent un train d'engrenage 38' comprenant, tout d'abord, deux pignons tronconiques assurant un renvoi d'angle et, ensuite, des pignons droits assurant l'entraînement ensemble mais, à des vitesses différentes des rouleaux de massage 37 et 38. Les moyens de manœuvre 38' sont de préférence adaptés pour assurer une vitesse de rotation du premier rouleau 37 supérieure à celle du deuxième rouleau 38 et dans le cas présent triple de celle du deuxième rouleau 38.

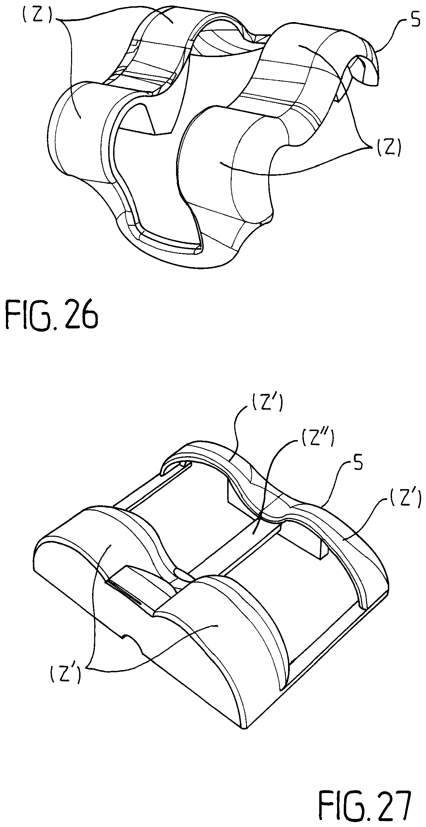

Par ailleurs selon cette variante, la tête de soin 7 comprend des LED à proximité des zones de contact (Z). Plusieurs agencements des LED pourraient être envisagés étant donné que les zones de contact (Z) sont couvertes chacune par un guide de lumière 5. Dans l'exemple illustré, deux LED sont agencées en haut de la tête de soin 7, c'est-à-dire à l'extrémité des zones de contact (Z) éloignée du corps 2 ; deux autres LED sont agencées en bas de la tête de soin 7, c'est-à-dire à l'autre extrémité des zones de contact (Z). La lumière est alors transmise par le guide de lumière 5 qui est présenté à la figure 26. Le guide de lumière selon cet exemple présente une forme de coque monobloc d'épaisseur sensiblement constante et épousant la forme de la zone de contact (Z). On pourrait également envisager un agencement des LED au milieu des zones de contact (Z) pour renforcer l'éclairage entre les deux rouleaux. Le guide de lumière pourrait alors avoir une épaisseur au milieu plus importante que celle à ses extrémités.

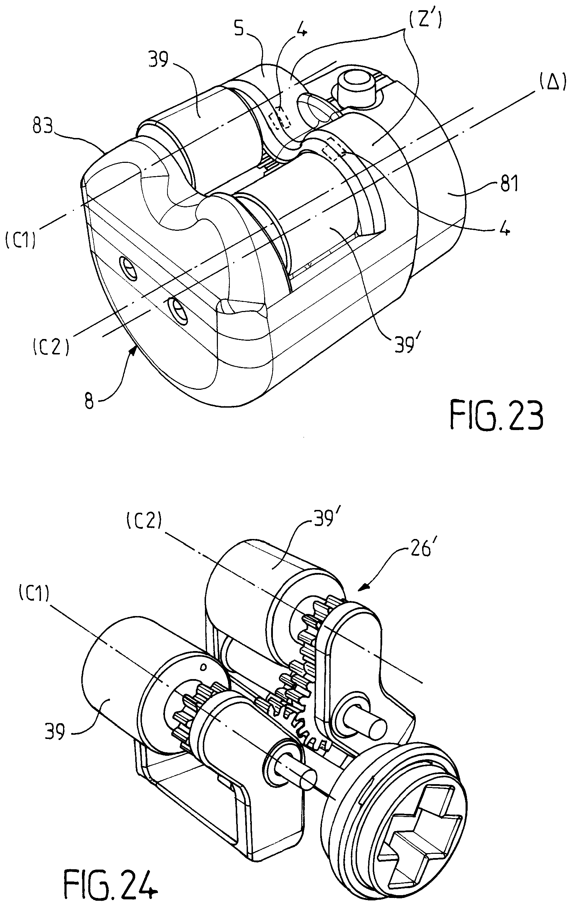

Un tel moyen de massage permet de masser la peau par léger pincement de manière à reproduire un massage de type « pincé Jacquet » réalisé par les professionnels de l'esthétique au moyen du pouce et de l'index de manière délicate. Ces effets sont accentués par la présence de la lumière sur la peau. La tête de soin 8 selon la quatrième variante telle qu'illustrée aux figures 23 à 25 est conçue pour fournir un massage du type « palper-rouler » sur la peau afin d'obtenir un effet remodelant. Une telle tête de soin 8 comprend une chambre à rouleaux 83 présentant une surface d'application (Z') destinée à venir en appui contre la peau et deux rouleaux cylindriques 39, 39' dont une partie dépasse de la surface d'application (Z'). Ces rouleaux cylindriques 39, 39' sont disposés selon deux axes C1, C2 parallèles entre eux et par rapport à l'axe central (Δ). Tel que visible à la figure 24, la tête de soin 8 comprend des moyens de manœuvre 26' comprenant un disque entrainé en rotation par l'arbre de sortie 25, le disque étant lié en rotation à une roue dentée. Les moyens de manœuvre 26' comprennent encore un train d'engrenages reliant la roue dentée à deux pignons liés en rotation respectivement avec chacun des deux rouleaux cylindriques 39, 39' et destinés à entraîner ceux-ci.

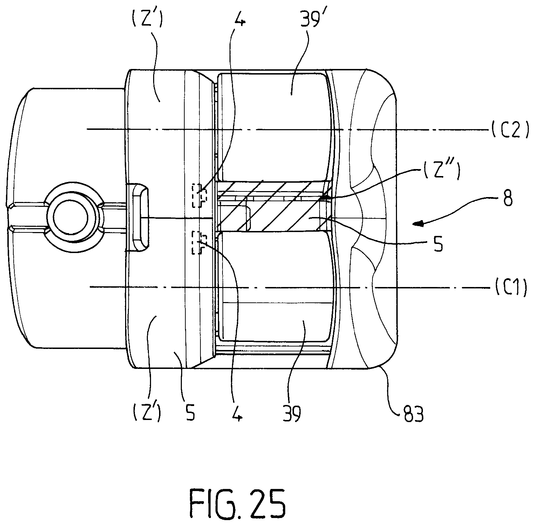

Selon l'invention et tel qu'illustré aux figures 23 et 25, la tête de soin 8 comprend en outre des LED 4 agencées à l'une des extrémités des rouleaux cylindriques 39, 39', préférablement à l'extrémité proximale au corps 2 de l'appareil. Pour mieux projeter la lumière provenant des LED, la tête de soin 8 est équipée d'un guide de lumière 5 présenté sous forme d'une coque monobloc ayant une forme en « I » vue de face. Le guide de lumière 5 comprend alors au moins une première partie recouvrant ladite surface d'application (Z') pour former une première zone d'éclairage (Z') et une deuxième partie recouvrant une surface au fond de la chambre à rouleaux 83, notamment celle entre les deux rouleaux cylindriques 39, 39' pour former une deuxième zone d'éclairage (Z").Le guide de lumière 5 selon cet exemple est illustré à la figure 27.

Un tel moyen de massage permet d'optimiser le travail de la peau par plissage de façon complète, agréable et efficace. Ces effets sont accentués par la présence de la lumière sur la peau.

Dans le but d'encore mieux transmettre la lumière vers la peau, les moyens de massage 31, 32, 33, 35, 36, 37, 37', 38, 39 et 39' peuvent être transparents ou translucides.

Selon les exemples illustrés, les LED sont intégrées dans la tête de soin 6, 7, 8, 9 ayant une fonction massage pour être au plus près de la peau. Cependant, il est tout à fait envisageable d'agencer les LED dans le corps 2 de l'appareil, à l'extrémité où est montée chaque tête de soin 6, 7, 8, 9. Ainsi on simplifie les connectiques électriques ce qui permet de rendre l'appareil plus sécurisant, économique et compact.

Bien entendu, l'invention n'est nullement limitée aux modes de réalisation décrits et illustrés qui n'ont été donnés qu'à titre d'exemple. Des modifications restent possibles, notamment du point de vue de la constitution des divers éléments ou par substitution d'équivalents techniques, sans sortir pour autant du domaine de protection de l'invention tel que défini dans les revendications attenantes.

## Revendications

1. Appareil de soin (1) pour la peau comprenant :
- un corps (2) comportant un boîtier (21, 22) formant une zone de préhension,
- des moyens d'alimentation électrique (23) agencés dans ledit corps,
- au moins une tête de soin (3, 6, 7, 8, 9),
- au moins une source lumineuse (4),
- au moins un guide de lumière (5) présentant une surface de projection (51) destinée à recevoir de la lumière émise par ladite au moins une source lumineuse (4) et à projeter au moins un faisceau lumineux à destination d'une zone sur la peau, ledit au moins un guide de lumière (5) comprenant une face opposée (52) à ladite surface de projection (51), ladite face opposée (52) comprenant des moyens de distribution (53) destinés à distribuer la lumière le long de ladite surface de projection (51),
le guide de lumière (5) étant fait en une pièce monobloc présentant à la fois la surface de projection (51) et la face opposée (52) qui est en regard, et lesdits moyens de distribution (53) comprenant au moins une surface (S1, S2) au moins partiellement réfléchissante destinées à réfléchir une partie de la lumière vers ladite surface de projection (51),
ledit au moins un guide de lumière (5) présentant au moins une section transversale (S) de passage de la lumière qui est une section passant par un plan (P) perpendiculaire à ladite surface de projection (51), ladite section transversale (S) étant sécante avec la surface de projection (51) et ladite au moins une surface (S1) respectivement suivant une première droite (D1) et une deuxième droite (D2), ladite au moins une source lumineuse (4) étant agencée à une extrémité de ladite section transversale (S) entre la première droite (D1) et la deuxième droite (D2),
**caractérisé en ce que** ledit au moins un guide de lumière (5) est un solide de révolution
de ladite section transversale (S) autour d'un axe central (Δ) et **en ce que** ladite au moins une source lumineuse (4) comprend une pluralité de diodes électroluminescentes (4) réparties de façon régulière autour dudit guide de lumière (5).

2. Appareil selon la revendication précédente **caractérisé en ce que** lesdits moyens de distribution (53) comprennent au moins deux surfaces (S1, S2) au moins partiellement réfléchissantes, dont une desdites deux surfaces (S2) et ladite section transversale (S) sont sécantes suivant une troisième droite (D3), la distance entre D1 et D2 étant supérieure à celle entre D1 et D3.

3. Appareil selon l'une des revendications précédentes **caractérisé en ce que** la face opposée (52) présente des cannelures (57) partant dudit axe central (Δ).

4. Appareil selon l'une des revendications précédentes **caractérisé en ce que** ladite face opposée (52) est au moins partiellement recouverte d'une couche de couleur blanche ou au moins partiellement recouverte d'une couche de couleur métallisée.

5. Appareil selon l'une des revendications précédentes **caractérisé en ce que** ladite surface de projection (51) présente des moyens diffusants tels que du grainage.

6. Appareil selon l'une des revendications précédentes **caractérisé en ce que** ladite au moins une tête de soin (3, 6, 7, 8, 9) comprend des moyens de massage (31, 32, 33, 34, 35, 36, 37, 37', 38, 39, 39') mécanique destinés à venir en contact avec la peau et des moyens de manœuvre (26, 31', 32', 33', 34', 35', 36', 38', 26') desdits moyens de massage (31, 32, 33, 34, 35, 36, 37, 37' 38, 39, 39') actionnés par un moteur électrique (24) qui est relié auxdits moyens d'alimentation électrique (23).

7. Appareil selon la revendication précédente **caractérisé en ce que** lesdits moyens de massage (31, 32, 33, 34, 35, 36, 37, 37', 38, 39, 39') comprennent au moins une bille de massage (31, 32, 33) destinée à être déplacée en rotation selon au moins un axe de rotation vertical (A1, A2, A3) qui est perpendiculaire à la surface de projection (51), ledit guide de lumière présentant préférentiellement au moins un trou traversant (541, 542, 543) laissant passer ladite bille de massage (31, 32, 33).

8. Appareil selon la revendication 6 **caractérisé en ce que** lesdits moyens de massage (31, 32, 33, 35, 36, 37, 37', 38, 39, 39') comprennent un élément d'appui (34) destiné à venir contre le visage et deux doigts de massage (35, 36) destinés à venir au contact de la peau de façon alternative, ledit guide de lumière (5) étant avantageusement agencé à proximité dudit élément d'appui (34) et sensiblement parallèle à celui-ci et ladite source lumineuse (4) comprenant préférentiellement au moins une diode électroluminescente (4) entre lesdits doigts de massage (35, 36) et le corps (2).

9. Appareil selon la revendication 6 **caractérisé en ce que** lesdits moyens de massage (31, 32, 33, 34, 35, 36, 37, 37', 38, 39, 39') comprennent deux rouleaux (37, 38) de massage qui sont mobiles en rotation sur eux-mêmes selon deux axes de rotation horizontaux (B1, B2) parallèles entre eux et chacun perpendiculaire à l'axe du corps (2), un premier rouleau (37) comprenant au moins une palette (37') s'étendant radialement en saillie de la surface du premier rouleau (37) et le deuxième rouleau (38) présentant une surface de type lisse, lesdits rouleaux (37, 38) étant actionnés par des moyens de manœuvre (38').

10. Appareil selon la revendication précédente **caractérisé en ce que** ledit au moins un guide de lumière (5) est destiné à recouvrir au moins une zone de contact (Z) à l'extrémité des rouleaux (37, 38) destinée à venir en contact avec la peau, ladite source lumineuse (4) comprenant préférentiellement au moins une diode électroluminescente (4) à proximité de ladite zone de contact (Z).

11. Appareil selon la revendication 6 **caractérisé en ce que** lesdits moyens de massage (31, 32, 33, 34, 35, 36, 37, 37', 38, 39, 39') comprennent deux rouleaux cylindriques (39, 39') qui sont mobiles en rotation sur eux-mêmes selon deux axes de rotation (C1, C2) parallèles entre eux et par rapport à l'axe du corps (2).

12. Appareil selon la revendication précédente **caractérisé en ce que** ledit au moins un guide de lumière (5) est destiné à recouvrir au moins une première zone d'éclairage (Z') à l'une des extrémités des rouleaux cylindriques (39, 39') et/ou une deuxième zone d'éclairage (Z") située entre lesdits rouleaux cylindriques (39, 39'), ladite première zone d'éclairage (Z') étant destinée à venir en contact avec la peau, ladite source lumineuse (4) comprenant préférentiellement au moins une diode électroluminescente à proximité de ladite première zone d'éclairage (Z').

13. Appareil selon une des revendications 6 12 **caractérisé en ce que** lesdits moyens de massage (31, 32, 33, 34, 35, 36, 37, 37', 38, 39, 39') sont transparents ou translucides.

14. Appareil selon l'une des revendications précédentes **caractérisé en ce que** ladite au moins une tête de soin (3, 6, 7, 8, 9) est détachable dudit corps (2), ledit appareil comprenant préférentiellement au moins deux têtes de soin (3, 6, 7, 8, 9) différentes interchangeables sur le corps (2).

## Patentansprüche

1. Behandlungseinrichtung (1) für die Haut, umfassend:
- einen Körper (2), der ein Gehäuse (21, 22) umfasst, das einen Greifbereich bildet,
- elektrische Versorgungsmittel (23), die im Körper eingerichtet sind,
- mindestens einen Behandlungskopf (3, 6, 7, 8, 9),
- mindestens eine Lichtquelle (4),
- mindestens einen Lichtleiter (5), der eine Projektionsfläche (51) aufweist, die dazu vorgesehen ist, Licht, das von der mindestens einen Lichtquelle (4) emittiert wird, aufzunehmen und mindestens einen Lichtstrahl zu projizieren, der auf einen Bereich auf der Haut gerichtet ist, wobei der mindestens eine Lichtleiter (5) eine zur Projektionsfläche (51) entgegengesetzte Seite (52) umfasst, wobei die entgegengesetzte Seite (52) Verteilungsmittel (53) umfasst, die dazu vorgesehen sind, das Licht entlang der Projektionsfläche (51) zu verteilen,
wobei der Lichtleiter (5) aus einem einteiligen Stück gefertigt ist, das zugleich die Projektionsfläche (51) und die entgegengesetzte Seite (52), die gegenüberliegt, aufweist und wobei die Verteilungsmittel (53) mindestens eine mindestens teilweise reflektierende Fläche (S1, S2) umfassen, die dazu vorgesehen ist, einen Teil des Lichts zur Projektionsfläche (51) hin zu reflektieren,
wobei der mindestens eine Lichtleiter (5) mindestens einen querverlaufenden Abschnitt (S) zum Durchlassen von Licht umfasst, der ein Abschnitt ist, der durch eine senkrecht zur Projektionsfläche (51) verlaufende Ebene (P) ist, wobei der querverlaufende Abschnitt (S) die Projektionsfläche (51) und die mindestens eine Fläche (S1) entlang einer ersten Geraden (D1) bzw. einer zweiten Geraden (D2) schneidet, wobei die mindestens eine Lichtquelle (4) an einem Ende des querverlaufenden Abschnitts (S) zwischen der ersten Geraden (D1) und der zweiten Geraden (D2) eingerichtet ist,
**dadurch gekennzeichnet, dass** der mindestens eine Lichtleiter (5) ein Rotationskörper des querverlaufenden Abschnitts (S) um eine mittlere Achse (Δ) ist und dadurch, dass die mindestens eine Lichtquelle (4) eine Vielzahl von lichtemittierenden Dioden (4) umfasst, die auf regelmäßige Weise um den Lichtleiter (5) aufgeteilt sind.

2. Einrichtung nach dem vorstehenden Anspruch, **dadurch gekennzeichnet, dass** die Verteilungsmittel (53) mindestens zwei mindestens teilweise reflektierende Flächen (S1, S2) umfassen, wobei eine der zwei Flächen (S2) und der querverlaufende Abschnitt (S) sich entlang einer dritten Gerarden (D3) schneiden, wobei der Abstand zwischen D1 und D2 größer ist als jener zwischen D1 und D3.

3. Einrichtung nach einem der vorstehenden Ansprüche, **dadurch gekennzeichnet, dass** die entgegengesetzte Seite (52) Nuten (57) aufweist, die von der mittleren Achse (Δ) ausgehen.

4. Einrichtung nach einem der vorstehenden Ansprüche, **dadurch gekennzeichnet, dass** die entgegengesetzte Seite (52) mindestens teilweise von einer Schicht mit weißer Farbe bedeckt ist oder mindestens teilweise von einer Schicht mit metallisierter Farbe bedeckt ist.

5. Einrichtung nach einem der vorstehenden Ansprüche, **dadurch gekennzeichnet, dass** die Projektionsfläche (51) Diffusionsmittel, wie etwa Körnung, aufweist.

6. Einrichtung nach einem der vorstehenden Ansprüche, **dadurch gekennzeichnet, dass** der mindestens eine Behandlungskopf (3, 6, 7, 8, 9) mechanische Massagemittel (31, 32, 33, 34, 35, 36, 37, 37', 38, 39, 39'), die dazu vorgesehen sind, mit der Haut in Berührung zu kommen, und Betätigungsmittel (26, 31', 32', 33', 34', 35', 36', 38', 26') der Massagemittel (31, 32, 33, 34, 35, 36, 37, 37', 38, 39, 39') umfasst, die durch einen Elektromotor (24) angetrieben werden, der mit den elektrischen Versorgungsmitteln (23) verbunden ist.

7. Einrichtung nach dem vorstehenden Anspruch, **dadurch gekennzeichnet, dass** die Massagemittel (31, 32, 33, 34, 35, 36, 37, 37', 38, 39, 39') mindestens eine Massagekugel (31, 32, 33) umfassen, die dazu vorgesehen ist, entlang mindestens einer vertikalen Drehachse (A1, A2, A3), die senkrecht zur Projektionsfläche (51) ist, drehend bewegt zu werden, wobei der Lichtleiter vorzugsweise mindestens eine Durchgangsöffnung (541, 542, 543) aufweist, die die Massagekugel (31, 32, 33) durchlässt.

8. Einrichtung nach Anspruch 6, **dadurch gekennzeichnet, dass** die Massagemittel (31, 32, 33, 35, 36, 37, 37', 38, 39, 39') ein Auflageelement (34), das dazu vorgesehen ist, auf dem Gesicht anzuliegen, und zwei Massagefinger (35, 36) umfassen, die dazu vorgesehen sind, auf alternative Weise mit der Haut in Berührung zu kommen, wobei der Lichtleiter (5) vorteilhafterweise in der Nähe des Auflageelements (34) und im Wesentlichen parallel zu diesem eingerichtet ist und wobei die Lichtquelle (4) vorzugsweise mindestens eine lichtemittierende Diode (4) zwischen den Massagefingern (35, 36) und dem Körper (2) umfasst.

9. Einrichtung nach Anspruch 6, **dadurch gekennzeichnet, dass** die Massagemittel (31, 32, 33, 34, 35, 36, 37, 37', 38, 39, 39') zwei Massagerollen (37, 38) umfassen, die um sich selbst entlang von zwei horizontalen Drehachsen (B1, B2), die parallel zueinander und jeweils senkrecht zur Achse des Körpers (2) sind, drehbeweglich sind, wobei eine erste Rolle (37) mindestens eine Palette (37') umfasst, die sich radial aus der Fläche der ersten Rolle (37) vorspringend erstreckt, und wobei die zweite Rolle (38) eine Fläche vom glatten Typ aufweist, wobei die Rollen (37, 38) durch Betätigungsmittel (38') angetrieben werden.

10. Einrichtung nach dem vorstehenden Anspruch, **dadurch gekennzeichnet, dass** der mindestens eine Lichtleiter (5) dazu vorgesehen ist, mindestens einen Berührungsbereich (Z) am Ende der Rollen (37, 38) zu bedecken, der dazu vorgesehen ist, mit der Haut in Berührung zu kommen, wobei die Lichtquelle (4) vorzugsweise mindestens eine lichtemittierende Diode (4) in der Nähe des Berührungsbereichs (Z) umfasst.

11. Einrichtung nach Anspruch 6, **dadurch gekennzeichnet, dass** die Massagemittel (31, 32, 33, 34, 35, 36, 37, 37', 38, 39, 39') zwei zylindrische Rollen (39, 39') umfassen, die um sich selbst entlang von zwei Drehachsen (C1, C2), die parallel zueinander und in Bezug auf die Achse des Körpers (2) sind, drehbeweglich sind.

12. Einrichtung nach dem vorstehenden Anspruch, **dadurch gekennzeichnet, dass** der mindestens eine Lichtleiter (5) dazu vorgesehen ist, mindestens einen ersten Beleuchtungsbereich (Z') an einem der Enden der zylindrischen Rollen (39, 39') und/oder einen zweiten Beleuchtungsbereich (Z"), der sich zwischen den zylindrischen Rollen (39, 39') befindet, zu bedecken, wobei der erste Beleuchtungsbereich (Z') dazu vorgesehen ist, mit der Haut in Berührung zu kommen, wobei die Lichtquelle (4) vorzugsweise mindestens eine lichtemittierende Diode in der Nähe des ersten Beleuchtungsbereichs (Z') umfasst.

13. Einrichtung nach einem der Ansprüche 6 bis 12, **dadurch gekennzeichnet, dass** die Massagemittel (31, 32, 33, 34, 35, 36, 37, 37', 38, 39, 39') durchsichtig oder lichtdurchlässig sind.

14. Einrichtung nach einem der vorstehenden Ansprüche, **dadurch gekennzeichnet, dass** der mindestens eine Behandlungskopf (3, 6, 7, 8, 9) vom Körper (2) abnehmbar ist, wobei die Einrichtung vorzugsweise mindestens zwei unterschiedliche, auswechselbare Behandlungsköpfe (3, 6, 7, 8, 9) auf dem Körper (2) umfasst.

## Claims

1. Apparatus (1) for treating skin comprising:
- a body (2) comprising a casing (21, 22) forming a grippable region,
- electrical means for supplying power (23), arranged in said body,
- at least one treatment head (3, 6, 7, 8, 9),
- at least one light source (4),
- at least one light guide (5) having a projection area (51) that is intended to receive the light emitted by said at least one light source (4) and to project at least one light beam intended for a region of skin, said at least one light guide (5) comprises a face opposite (52) said projection area (51), said opposite face (52) comprising distributing means (53) intended to distribute the light over said projection area (51),
the light guide (5) being made of a one-piece part having both the projection area (51) and the opposite face (52) which is facing it, and said distribution means (53) comprising at least one area (S1, S2) at least partially reflective intended to reflect some of the light towards said projection area (51),
said at least one light guide (5) having at least one transversal cross-section (S) for passing the light which is a cross-section passing through a plane (P) perpendicular to said projection area (51), said transversal cross-section (S) being secant with the projection area (51) and said at least one area (S1) respectively following a first straight line (D1) and a second straight line (D2), said at least one light source (4) being arranged at an end of said transversal cross-section (S) between the first straight line (D1) and the second straight line (D2),
**characterised in that** said at least one light guide (5) is a revolving solid of said transversal cross-section (S) about a central axis (Δ) and **in that** said at least one light source (4) comprises a plurality of light-emitting diodes (4) distributed regularly about said light guide (5).

2. Apparatus according to the preceding claim, **characterised in that** said distribution means (53) comprise at least two areas (S1, S2) at least partially reflective, of which one of said two areas (S2) and said transversal cross-section (S) are secant following a third straight line (D3), the distance between D1 and D2 being greater than that between D1 and D3.

3. Apparatus according to one of the preceding claims, **characterised in that** the opposite face (52) has slots (57) starting from said central axis (Δ).

4. Apparatus according to one of the preceding claims, **characterised in that** said opposite face (52) is at least partially covered with a white-coloured layer or at least partially covered with a metallic-coloured layer.

5. Apparatus according to one of the preceding claims, **characterised in that** said projection area (51) has diffusing means such as roughening means.

6. Apparatus according to one of the preceding claims, **characterised in that** said at least one treatment head (3, 6, 7, 8, 9) comprises mechanical massage means (31, 32, 33, 34, 35, 36, 37, 37', 38, 39, 39') intended to come into contact with the skin and means for manoeuvring (26, 31', 32', 33', 34', 35', 36', 38', 26') said massage means (31, 32, 33, 34, 35, 36, 37, 37', 38, 39, 39') actuated by an electric motor (24) which is connected to said electrical power means (23).

7. Apparatus according to the preceding claim, **characterised in that** said massage means (31, 32, 33, 34, 35, 36, 37, 37', 38, 39, 39') comprise at least one massage ball (31, 32, 33) intended to be displaced in rotation according to at least one vertical axis of rotation (A1, A2, A3) which is perpendicular to the projection area (51), said light guide preferably having at least one through hole (541, 542, 543) letting said massage ball (31, 32, 33) pass through.

8. Apparatus according to claim 6, **characterised in that** said massage means (31, 32, 33, 35, 36, 37, 37', 38, 39, 39') comprise a support element (34) intended to come against the face and two massage fingers (35, 36) intended to come into contact with the skin alternatively, said light guide (5) being advantageously arranged in the proximity of said support element (34) and substantially parallel to this and said light source (4) preferably comprising at least one light-emitting diode (4) between said massage fingers (35, 36) and the body (2).

9. Apparatus according to claim 6, **characterised in that** said massage means (31, 32, 33, 34, 35, 36, 37, 37', 38, 39, 39') comprise two massage rollers (37, 38) which are movable in rotation on themselves along two horizontal axes of rotation (B1, B2) parallel to one another and each perpendicular to the axis of the body (2), a first roller (37) comprising at least one palette (37') extending radially projecting from the area of the first roller (37) and the second roller (38) having a smooth-type surface, said rollers (37, 38) being actuated by manoeuvring means (38').

10. Apparatus according to the preceding claim, **characterised in that** said at least one light guide (5) is intended to cover at least one contact region (Z) at the end of the rollers (37, 38) intended to come into contact with the skin, said light source (4) preferably comprising at least one light-emitting diode (4) in the proximity of said contact region (Z).

11. Apparatus according to claim 6, **characterised in that** said massage means (31, 32, 33, 34, 35, 36, 37, 37', 38, 39, 39') comprise two cylindrical rollers (39, 39') which are movable in rotation on themselves along two axes of rotation (C1, C2) parallel to one another and with respect to the axis of the body (2).

12. Apparatus according to the preceding claim, **characterised in that** said at least one light guide (5) is intended to cover at least one first lighting region (Z') at one of the ends of the cylindrical rollers (39, 39') and/or a second lighting region (Z") located between said cylindrical rollers (39, 39'), said first lighting region (Z') being intended to come into contact with the skin, said light source (4) preferably comprising at least one light-emitting diode in the proximity of said first lighting region (Z').

13. Apparatus according to one of claims 6 to 12, **characterised in that** said massage means (31, 32, 33, 34, 35, 36, 37, 37', 38, 39, 39') are transparent or translucid.

14. Apparatus according to one of the preceding claims, **characterised in that** said at least one treatment head (3, 6, 7, 8, 9) is detachable from said body (2), said apparatus preferably comprising at least two different treatment heads (3, 6, 7, 8, 9) which are interchangeable on the body (2).
